(19) Europäisches Patentamt
European Patent Office
Office européen des brevets

(11) Veröffentlichungsnummer: **0 055 387**
**B1**

# (12) EUROPÄISCHE PATENTSCHRIFT

(45) Veröffentlichungstag der Patentschrift: **16.07.86**

(51) Int. Cl.⁴: **C 07 D 301/14** // C07D303/04

(21) Anmeldenummer: **81109418.4**

(22) Anmeldetag: **30.10.81**

## (54) Verfahren zur Herstellung von Epoxiden.

(30) Priorität: **30.12.80 DE 3049434**

(43) Veröffentlichungstag der Anmeldung: **07.07.82 Patentblatt 82/27**

(45) Bekanntmachung des Hinweises auf die Patenterteilung: **16.07.86 Patentblatt 86/29**

(84) Benannte Vertragsstaaten: **AT BE CH DE FR GB IT LI NL**

(56) Entgegenhaltungen:
**DE - A - 2 602 776**
**DE - A - 2 619 091**
**DE - A - 2 835 940**

**THE JOURNAL OF THE AMERICAN CHEMICAL SOCIETY, Band 82, Juli-September 1960, Easton, Pa., M. KORACH et al. "Synthesis and Reactions of 3,4-Epoxycyclopentene", Seite 4328**

**Die Akte enthält technische Angaben, die nach dem Eingang der Anmeldung eingereicht wurden und die nicht in dieser Patentschrift enthalten sind.**

(73) Patentinhaber: **Peroxid-Chemie GmbH, Dr.-Gustav-Adolph-Strasse 2, D-8023 Höllriegelskreuth bei München (DE)**

(72) Erfinder: **Edl, Wolfgang, Dr., Marienstrasse 3, D-8023 Grosshesselohe (DE)**
Erfinder: **Sienel, Günter R., Dr., Anwänden 5, D-8026 Ebenhausen (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

**EP 0 055 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Epoxiden, insbesondere zur Herstellung von säurelabilen Epoxiden.

Epoxide (Oxirane) sind wertvolle Zwischenprodukte, z.B. zur Herstellung von Riechstoffen. Einige Terpenepoxide finden auch als Riechstoffe Verwendung.

Das klassische Verfahren zur Herstellung von Epoxiden ist die sogenannte PRILESCHAJEW-Reaktion, d.h. die Epoxidation von Olefinen mittels Percarbonsäure (vgl. D. Swern, Organic Peroxides, Vol. II. Wiley-Interscience, 1971, 355–533).

Für Epoxidationen im Labormassstab wird neuerdings häufig die relativ stabile, käufliche m-Chlorperbenzoesäure verwendet, die jedoch für den industriellen Massstab aus Kostengründen nicht in Frage kommt. Von technischem Interesse sind insbesondere die niederen aliphatischen Gleichgewichts-Percarbonsäuren mit 2–4 C-Atomen, die durch Umsetzung der entsprechenden Carbonsäure mit (möglichst hochprozentigem) Wasserstoffperoxid in Gegenwart einer geringen Menge starker Säure als Katalysator gemäss der folgenden Gleichung entstehen.

$$RCOOH + H_2O_2 \xrightleftharpoons{[H^+]} RCO{-}O{-}O{-}H + H_2O$$

Gleichgewichts-Percarbonsäuren stellen also ein Gemisch aus Percarbonsäure, Carbonsäure, Wasserstoffperoxid, Wasser und starker Säure dar. Die Zusammensetzung hängt ab vom Molverhältnis Carbonsäure/Wasserstoffperoxid und von der Konzentration des eingesetzten Wasserstoffperoxids. Als starke Säure wird vorzugsweise Schwefelsäure in einer Menge von 0,5–1% verwendet.

Die bevorzugte Percarbonsäure ist Peressigsäure, wobei insbesondere die handelsübliche Gleichgewichts-Peressigsäure (kurz PES-40 genannt) mit folgender ungefährer Zusammensetzung eingesetzt wird:

40% Peressigsäure
40% Essigsäure
5% Wasserstoffperoxid
14% Wasser
1% $H_2SO_4$

Es sind aber auch verdünntere Systeme anwendbar.

Perpropionsäure oder Perbuttersäuren sind insbesondere dann angezeigt, wenn dadurch eine destillative Aufarbeitung im Hinblick auf die Siedepunkte erleichtert oder erst ermöglicht wird.

Die weiteren Ausführungen werden unter bezug auf das System PES-40 gemacht. Für verdünntere Systeme und analoge Systeme anderer niederer Percarbonsäuren gelten jedoch sinngemäss die gleichen Prinzipien.

Die Herstellung vieler Epoxide mit PES-40 in hoher Ausbeute und Reinheit scheitert daran, dass das zunächst gebildete Epoxid in einer Folgereaktion mit Essigsäure bzw. Wasser unter Ringöffnung und Bildung des entsprechenden Hydroxyacetats oder vicinalen Diols reagiert. Diese Reaktionen sind säurekatalysiert, so dass die Anwesenheit von Schwefelsäure besonders problematisch ist. Eine weitere unerwünschte Folgereaktion ist die säurekatalysierte Umlagerung des Epoxids zu Carbonylverbindungen.

Zur Ausschaltung des störenden Einflusses der Schwefelsäure wurde daher vorgeschlagen, vor der Zugabe der PES-40 zum Olefin, das gegebenenfalls in einem Lösungsmittel gelöst ist, mit einer anorganischen Base, z.B. Natriumacetat, Natriumhydrogencarbonat oder Calciumcarbonat zu neutralisieren (vgl. Swern, S. 436). Im Falle nicht zu reaktiver Epoxide lassen sich mit dieser sogenannten «gepufferten Gleichgewichts-Percarbonsäure» gute Ergebnisse erzielen.

Bei besonders säurelabilen Epoxiden reicht diese Massnahme nicht aus. Hier genügt offensichtlich schon die Anwesenheit der relativ grossen Menge an Essigsäure, die mit PES-40 eingeführt und bei der Reaktion entsteht, um Ringöffnung zu bewirken. Zusatz von schwachen Basen, wie Natriumcarbonat und Natriumhydrogencarbonat, in einer Menge, die ausreicht, um die gesamte Mineralsäure und zumindest den grössten Teil der Essigsäure zu neutralisieren, zu dem Reaktionsgemisch ermöglicht in solchen Fällen eine Epoxidation (vgl. Swern, Seite 399, 436; Beispiele: Seite 423 und 424). Dieses Verfahren macht von der Tatsache Gebrauch, dass Essigsäure (in Methylenchlorid) ca. 2000-mal rascher neutralisiert wird als Peressigsäure (vgl. M. Korach et al., J. Am. Chem. Soc. 82, (1960), 4328).

Die DE-OS 2 835 940 schlägt für die Herstellung von reaktiven bicyclischen Terpen-Epoxiden, z.B. α-Pinen-oxid, mit PES-40 ebenfalls das vorstehend erläuterte sogenannte «Soda-Verfahren» vor. Zur Erzielung brauchbarer Ergebnisse ist allerdings noch die Verwendung bestimmter chlorierter Kohlenwasserstoffe erforderlich. Eine Nacharbeitung der Beispiele der DE-OS 2 835 940 ergab jedoch sehr schwankende Ergebnisse hinsichtlich Ausbeute und Produktreinheit. Es wurde eine deutliche Abhängigkeit von den Rührbedingungen und der Beschaffenheit der festen Base hinsichtlich Kornverteilung und Oberflächenstruktur festgestellt, so dass die Reproduzierbarkeit des Verfahrens nicht gegeben ist.

Aufgabe der vorliegenden Erfindung war es deshalb, die aufgezeigten Nachteile zu überwinden und ein Verfahren bereitzustellen, das eine Epoxidierung mit hoher Ausbeute und hoher Produktreinheit unter jederzeit reproduzierbaren Bedingungen ermöglicht.

Es wurde nun überraschenderweise gefunden, dass die aufgezeigten Nachteile dadurch überwunden werden können, dass man die in der Percarbonsäure vorhandene starke Säure neutralisiert, bevor sie zu dem Reaktionsgemisch aus Olefin, säurebindendem Mittel und Lösungsmittel zugefügt wird. Dabei wurde auch festgestellt, dass auf diese Weise die erforderliche Menge an säurebindendem Mittel, wie z.B. Alkalicarbonat oder -hydrogencarbonat, deutlich reduziert werden kann.

Gegenstand der Erfindung ist ein Verfahren zur Herstellung von Epoxiden durch Epoxidation der zugrundeliegenden Olefine mit starke Säuren enthaltender Peressigsäure in Gegenwart von säurebindenden Mitteln in einem Lösungsmittel und/oder Überschuss des Olefins, welches dadurch gekennzeichnet ist, dass die starke Säure vor der Zugabe der Peressigsäure zu dem Reaktionsgemisch mit einer mindestens äquivalenten Menge einer anorganischen Base gebunden wird und dass das Reaktionsgemisch neben dem entsprechenden Olefin noch 0,50 bis 0,74 Val säurebindendes Mittel pro Mol Gesamtmenge an Peressigsäure und Essigsäure enthält.

Säurelabile Epoxide sind solche, die in Gegenwart niederer Carbonsäuren Veränderungen erleiden. Sie leiten sich insbesondere ab von Olefinen, die an mindestens einem C-Atom der olefinischen Doppelbindung zwei Substituenten oder einen Arylrest tragen, oder von Cycloolefinen, insbesondere von den bicyclischen Terpenen. Beispiele für derartige Olefine sind Isobuten, 1,1,2-Trimethyläthylen, Tetramethyläthylen, Styrol, Acenaphthylen, Cyclohexen, 1-Alkylcyclohexen, alpha- und β-Pinen, Camphen, Linalylacetat, Cedren und Calaren.

Als Epoxidierungsagens dienen vorzugsweise Lösungen der Peressigsäure in der zugrundeliegenden Carbonsäure, die daneben noch eine starke Säure enthalten. Bevorzugte derartige Lösungen sind die oben beschriebenen sogenannten Gleichgewichts-Peressigsäuren, die durch säurekatalysierte Umsetzung der Essigsäure mit Wasserstoffperoxid gebildet werden. Besonders bevorzugt ist dabei die Gleichgewichts-Peressigsäure (PES-40).

Der Term «Gleichgewichts-Peressigsäure» ist dabei nicht einengend dahingehend zu verstehen, dass ein echtes Gleichgewicht eingestellt sein muss. Vielmehr wird dadurch zum Ausdruck gebracht, dass neben der Peressigsäure noch Essigsäure und katalytische Mengen einer starken Säure vorliegen, sowie eventuell noch Wasser und Wasserstoffperoxid. Dementsprechend können auch echte Gleichgewichtssysteme, die nachträglich modifiziert wurden, z.B. durch Zugabe des entsprechenden Carbonsäureanhydrids oder durch Zugabe eines inerten Verdünnungsmittels, erfindungsgemäss verwendet werden.

Als starke Säure liegt in den Gleichgewichts-Percarbonsäuren gewöhnlich Schwefelsäure in einer Menge von 0,5 bis 1% vor. Aber auch andere starke Mineralsäuren, wie Phosphor- und Salpetersäure, und starke organische Säuren, z.B. Alkansulfonsäuren, sind verwendbar.

Als säurebindendes Mittel zur Neutralisation der Essigsäure werden vorzugsweise Alkalicarbonate oder Alkalihydrogencarbonate, insbesondere Natriumcarbonat oder Natriumhydrogencarbonat verwendet. Deren Menge richtet sich nach der Empfindlichkeit des Epoxids gegenüber der Essigsäure. Im Regelfall ist sie so zu bemessen, dass die usprünglich vorhandene Essigsäure vollständig neutralisiert wird. Bei weniger labilen Epoxiden genügen bereits ca. 75% dieser Menge. Im Extremfall muss auch die aus der Peressigsäure gebildete Essigsäure gebunden werden. Die Zugabe des säurebindenden Mittels erfolgt in fester Form.

Die Umsetzung wird in Gegenwart eines Lösungsmittels und/oder eines Überschusses des Olefins durchgeführt, wobei die Menge ausreichend sein muss, um die Rührfähigkeit des Reaktionsgemisches zu gewährleisten. Halogen-, insbesondere Chlorkohlenwasserstoffe, wie Methylenchlorid, Chloroform, 1,2-Dichloräthan, 1,2-Dichlorpropan, Chlorbenzol sind (aus kinetischen Gründen; Swern, S. 459) die bevorzugten Lösungsmittel. Jedoch sind auch andere inerte Lösungsmittel, wie Benzol, Toluol, Äthylacetat, Dioxan, verwendbar, allerdings bei entsprechend längerer Reaktionszeit. Insbesondere ist mit dem erfindungsgemässen Verfahren keine so starke Abhängigkeit von Lösungsmitteln erforderlich wie nach dem Verfahren der DE-OS 2 835 940, wonach einigermassen zufriedenstellende Resultate nur mit bestimmten Chlorkohlenwasserstoffen als Lösungsmittel erreicht werden können.

Die Reaktionstemperatur richtet sich im allgemeinen nach der Reaktivität des Olefins; sie liegt üblicherweise zwischen −20 und 60 °C, vorzugsweise bei 10 bis 40 °C. Höhere Temperaturen sollten vermieden werden, um die Zersetzung der Percarbonsäure möglichst gering zu halten. Zu diesem Zweck können auch bekannte Peroxid-Stabilisatoren, wie z. B. 2,6-Dipicolinsäure und Hydroxyäthandiphosphonsäure, zugesetzt werden.

Anorganische Basen zur Bindung der starken Säure können sein die Alkali- oder Erdalkalisalze der jeweiligen Carbonsäure oder die Carbonate, Hydrogencarbonate, Oxide oder Hydroxide der Alkali- oder Erdalkalimetalle. Sie werden bevorzugt in fester Form zur Lösung der Peressigsäure zugefügt; es können aber auch wässrige, vorzugsweise konzentrierte wässrige Lösungen zur Anwendung kommen. Bei der Zugabe der anorganischen Base zur Lösung der Peressigsäure sich eventuell bildende Niederschläge können, falls Dosierungsprobleme zu befürchten sind, abfiltriert oder durch Zufügen einer geringen Menge Wasser in Lösung gebracht werden.

Die Auswahl unter den erfindungsgemäss einzusetzenden anorganischen Basen ist unkritisch; mildere Basen, wie die Carboxylate und Carbonate, haben den Vorteil, dass selbst bei Überdosierung eine allzu starke Alkalisierung vermieden wird, was eine Destabilisierung der Percarbonsäure zur Folge hätte. Natriumacetat, wasserfrei oder als Trihydrat, ist besonders geeignet. Ein weiteres Auswahlkriterium ist ein möglichst geringer Gehalt an Schwermetallionen, die ebenfalls eine Destabilisierung bewirken können. Es kann daher zweckmässig sein, der wässrigen Lösung der anorganischen Base und/oder der Lösung der Peressigsäure zur Bindung störender Schwermetallionen geeignete Maskierungsmittel, wie insbesondere Chelatbildner für Schwermetallionen, zusetzen.

Die nachfolgenden Beispiele erläutern die Erfindung näher, ohne sie darauf zu beschränken.

Durch die beiden Vergleichsbeispiele 1 und 2 wird ausserdem der überraschende technische Fortschritt des erfindungsgemässen Verfahrens gegenüber den Verfahren des Standes der Technik veranschaulicht.

Prozentangaben beziehen sich vorstehend und nachstehend auf Gewichtsprozente.

Beispiele:

Bei allen Beispielen wurde handelsübliche Gleichgewichts-Peressigsäure (im folgenden mit PES-40 abgekürzt) der folgenden Zusammensetzung verwendet:

| | |
|---|---|
| PES (Peressigsäure) | : 38,6% |
| Essigsäure | : 44,5% |
| Wasserstoffperoxid | : 3,8% |
| Wasser | : 12 % |
| Schwefelsäure | : 1 % |

Vergleichsbeispiel 1
(gemäss DE-OS 2 835 940)

In einem 1 l-Kolben, der mit Rührer, Innenthermometer, Rückflusskühler und Tropftrichter ausgerüstet ist, werden 75,7 g (0,5 Mol) 90%iges α-Pinen, 187 g Chloroform und 79,8 g (0,75 Mol) wasserfreies Natriumcarbonat vorgelegt und auf 35° erwärmt. Anschliessend tropft man 116,3 g (0,59 Mol) PES-40 unter Kühlung innerhalb 1,5 h so zu, dass die Reaktionstemperatur 40 °C beträgt.

Man lässt noch 1 h bei 40 °C rühren und gibt anschliessend zum Reaktionsgemisch 200 g Wasser, wobei das gebildete Natriumacetat in Lösung geht. Man trennt die organische Phase ab und wäscht sie nochmals mit 100 ml Wasser.

Nach dem Abdestillieren des Chloroforms im Vakuum am Rotationsverdampfer werden 89 g α-Pinenoxid mit einem Gehalt von 61% erhalten; dies entspricht einer Ausbeute von 71% d.Th., bezogen auf α-Pinen.

Vergleichsbeispiel 2
(mit «gepufferter Gleichgewichts-Peressigsäure»)

75,7 g (0,5 Mol) 90%iges α-Pinen werden in 187 g Chloroform gelöst und in der im Vergleichsbeispiel 1 beschriebenen Apparatur auf 25 °C erwärmt. Unter Kühlung tropft man im Verlauf von 1 h 116 g (0,59 Mol) PES-40, die zuvor unter Rühren mit 2 g (0,025 Mol) Natriumacetat versetzt wurde so zu, dass eine Reaktionstemperatur von 30 °C eingehalten wird. Nach 2 h Nachreaktionszeit bei 30 °C wird das gebildete Natriumacetat in 200 g Wasser gelöst und die organische Phase gaschromatographisch, unter Lösungsmittelunterdrückung, analysiert. Das Reaktionsgemisch besteht aus 8,5% α-Pinenoxid, 17,2% nicht umgesetztem α-Pinen und 74,3% Nebenprodukten.

Dieses Beispiel zeigt, dass α-Pinenoxid unter den angegebenen Reaktionsbedingungen nicht stabil ist.

Beispiel 1

75,7 g (0,5 Mol) 90%iges α-Pinen, 187 g Chloroform und 42,4 g (0,4 Mol) wasserfreies Natriumcarbonat werden in einem mit Rührer, Rückflusskühler, Innenthermometer und Tropftrichter versehenen 1 l-Kolben vorgelegt und auf 25 °C erwärmt. Anschliessend lässt man 116 g (0,59 Mol) PES-40, der vor der Zugabe unter Rühren 2 g (0,025 Mol) Natriumacetat zugesetzt wurde, unter Solekühlung im Verlauf von 1 h so zutropfen, dass die Reaktionstemperatur 30 °C beträgt. Anschliessend rührt man 2 h bei 30 °C nach.

Durch Zugabe von 200 g Wasser wird das entstandene Natriumacetat gelöst und nach erfolgter Phasentrennung mit 100 g 7%iger Natriumhydrogencarbonatlösung gewaschen. Nach Abziehen des Chloroforms am Rotationsverdampfer werden 76 g α-Pinenoxid mit einem Gehalt von 95% erhalten; dies entspricht einer Ausbeute von 95% d.Th., bezogen auf α-Pinen.

Beispiel 2

In einem 250 l Edelstahlreaktor, der mit einem Rückflusskühler, Dosiervorrichtung, Ankerrührer und Temperaturanzeige ausgestattet ist, werden 29,1 kg (211,4 Mol) 99%iges α-Pinen in 44,3 l Chloroform/24,5 kg wasserfreiem Natriumcarbonat durch Zugabe von 51 kg (258,7 Mol) PES-40, in der zuvor 853 g Natriumacetat gelöst wurden, bei 28–35 °C zur Reaktion gebracht.

Nach einer Zutropfzeit von 1,5 h wird weitere 1,5 h bei 28–35 °C nachgeführt.

Aufarbeitung und Isolierung erfolgen entsprechend den im Ausführungsbeispiel 1 genannten Bedingungen.

Ergebnis:

Es wurden 31,2 kg α-Pinenoxid, entsprechend einer Ausbeute von 94% erhalten. Die gaschromatographisch durchgeführte Analyse ergibt einen Gehalt von 97% mit 0,2% Chloroform- und < 0,2% α-Pinen-Anteilen.

Dieses Beispiel zeigt, dass die erfindungsgemässe Reaktion ohne Schwierigkeiten in einen grösseren Massstab übertragen werden kann.

Beispiel 3

1032 g (5,0 Mol) 99%iges α-Cedren, 2271 g Chloroform und 532 g (5,0 Mol) wasserfreies Natriumcarbonat werden in einem mit Rührer, Rückflusskühler, Innenthermometer und Tropftrichter versehenen 10 l-Glasreaktor vorgelegt und auf 15 °C erwärmt. Anschliessend lässt man 1183 g (6,0 Mol) PES-40, der vor der Zugabe 21 g (0,25 Mol) Natriumacetat zugesetzt wurde, im Verlauf von 1 h so zutropfen, dass die Reaktionstemperatur mit Hilfe von Solekühlung 20 °C beträgt. Anschliessend rührt man 3 h bei 20 °C nach.

Durch Zugabe von 1750 g Wasser wird das entstandene Natriumacetat gelöst und nach der Phasentrennung mit je 1000 g 7%iger Natriumhydrogencarbonatlösung und Wasser gewaschen.

Nach Abziehen des Chloroforms am Rotationsverdampfer werden 1114 g α-Cedrenoxid mit einem Gehalt von 94% erhalten; dies entspricht einer Ausbeute von 95% d.Th., bezogen auf α-Cedren.

Beispiel 4

991 g (5,0 Mol) 99%iges Linalylacetat, 2180 g Chloroform und 532 g (5,0 Mol) wasserfreies Natriumcarbonat werden in einem 10 l Glasreaktor mit 1183 g (6,0 Mol) PES-40, der 21 g (0,25 Mol) Natriumacetat zugesetzt wurde, unter Zutropfen innerhalb 1 h bei 20 °C zur Reaktion gebracht. Man lässt 2 h bei 20 °C nachreagieren und löst das gebildete Natriumacetat durch Zugabe von 1750 g Wasser.

Die organische Phase wird mit je 1000 g 7%iger Natriumhydrogencarbonatlösung und Wasser gewaschen und anschliessend das Chloroform am Rotationsverdampfer im Wasserstrahlvakuum bei 50 °C abdestilliert.

Die Ausbeute an Epoxylinalylacetat beträgt 1072 g mit einem Gehalt von 99% und ist quantitativ.

Beispiel 5

83 g (1,0 Mol) 99%iges Cyclohexen, 332 g Dichlormethan und 106 g (1,0 Mol) wasserfreies Natriumcarbonat werden in einem 2 l Rundkolben, der mit Rührer, Rückflusskühler, Innenthermometer und Tropftrichter versehen ist, vorgelegt und auf 25 °C erwärmt. Im Verlauf von 1 h lässt man unter Kühlung 237 g (1,2 Mol) PES-40, der 4 g (0,05 Mol) Natriumacetat zugesetzt wurde, so zutropfen, dass eine Reaktionstemperatur von 30 °C eingehalten wird. Man rührt weitere 2 h bei 30 °C nach. Unter Zugabe von 300 g Wasser wird das entstandene Natriumacetat gelöst. Nach erfolgter Phasentrennung wird die Dichlormethanphase einmal mit 100 g 7%iger Natriumhydrogencarbonatlösung gewaschen und anschliessend der fraktionierten Vakuumdestillation unter Verwendung einer gut trennenden Kolonne unterworfen. Die erhaltene Cyclohexenoxidmenge beträgt 93 g und weist einen Gehalt von 99% auf; dies entspricht einer Ausbeute von 94% d.Th., bezogen auf Cyclohexen.

Beispiel 6

104 g (1,0 Mol) reines Styrol, 260 g Chloroform und 168 g (2,0 Mol) Natriumhydrogencarbonat werden in einem 2 l Rundkolben, ausgestattet mit Rührer, Rückflusskühler, Innenthermometer und Tropftrichter, vorgelegt, auf 35 °C erwärmt und anschliessend mit insgesamt 319 g (1,6 Mol) PES-40, der 8 g (0,1 Mol) Natriumacetat zugesetzt wurde, unter Zutropfen zur Reaktion gebracht.

Nach einer Reaktionszeit von 9 h wird in bekannter Weise aufgearbeitet.

Ergebnis:

113 g Styroloxid mit einem Gehalt von 95%; dies entspricht einer Ausbeute von 89% d.Th., bezogen auf Styrol.

Beispiel 7

Zu 66,6 g (1,18 Mol) kondensiertem, reinem Isobuten, 324 g Chloroform und 106 g (1,0 Mol) wasserfreiem Natriumcarbonat werden in einem 1 l-Doppelmantelreaktor bei 5–10 °C 277,8 g (1,4 Mol) PES-40, der 8 g (0,1 Mol) Natriumacetat zugesetzt wurde, getropft. Nach einer Reaktionszeit von 6 h zeigt ein angefertigtes Gaschromatogramm vollständigen Umsatz des Isobutens zum Isobutenoxid an.

Anschliessend wird in bekannter Weise – ohne Abdestillieren des Chloroforms – aufgearbeitet. Das Gaschromatogramm – unter Lösungsmittelunterdrückung angefertigt – zeigt einen Isobutenoxidgehalt von 99%.

Beispiel 8

Ansatz:

138 g (1,0 Mol) 99%iges α-Pinen
303 g Benzol
106 g (1,0 Mol) wasserfreies Natriumcarbonat
235 g (1,19 Mol) PES-40, enthaltend
4 g (0,05 Mol) Natriumacetat

Verfahren:

Durchführung und Bedingungen entsprechen Beispiel 1, jedoch mit einer abweichenden Reaktionszeit von 5 Stunden.

Ergebnis:

121 g α-Pinenoxid mit einem Gehalt von 97%, entsprechen einer Ausbeute von 77% der Theorie, bezogen auf α-Pinen. Die geringe Ausbeute ist auf eine unzureichende Trennung Benzol/α-Pinenoxid bei der destillativen Aufarbeitung am Rotationsverdampfer zurückzuführen. Im Destillat (Benzol) war α-Pinenoxid enthalten.

Beispiel 9

Zu 688 g (10,0 Mol) Cyclopenten, 2730 g Dichlormethan und 1060 g (10,0 Mol) wasserfreiem Natriumcarbonat in einem 10 l Glasreaktor lässt man 2366 g (12,0 Mol) PES-40, der 41 g (0,5 Mol) Natriumacetat zugesetzt wurde, im Verlauf von 1 h so zutropfen, dass die Reaktionstemperatur mit Hilfe von Solekühlung 30 °C beträgt. Anschliessend rührt man 3 h bei 30 °C nach.

Durch Zugabe von 4000 ml Wasser wird das entstandene Natriumacetat gelöst und nach der Phasentrennung mit 1000 ml Wasser gewaschen. Durch Rektifikation mit einer gut trennenden Kolonne werden 876 g Cyclopentenoxid mit einem Gehalt von 98% erhalten. Die Ausbeute ist quantitativ.

Beispiel 10

543 g (5,0 Mol) cis, cis-Cyclooctadien-1.5, 4345 g Chloroform und 1060 g (10,0 Mol) wasserfreies Natriumcarbonat werden in einem 10 l Glasreaktor mit insgesamt 2.563 g (13,0 Mol) PES-40, der 46 g (0,46 Mol) Natriumacetat zugesetzt wurde, unter 2-stündigem Zutropfen bei 20 °C zur Reaktion gebracht. Man lässt 3 h nachreagieren und löst das gebildete Natriumacetat durch Zugabe von 4000 ml Wasser.

Die organische Phase wird mit je 2000 g 7%iger Natriumhydrogencarbonatlösung und Wasser gewaschen und anschliessend das Chloroform am Rotationsverdampfer im Wasserstrahlvakuum bei 50 °C abdestilliert.

Es werden 690 g cis,cis-1.5-Diepoxycyclooctan mit einem Gehalt von 95% erhalten; dies entspricht einer Ausbeute von 94% d.Th., bezogen auf eingesetztes Olefin.

Beispiel 11

165 g (1,0 Mol) Cyclododecatrien-1.5.9, 500 g Chloroform und 106 g (1,0 Mol) wasserfreies Natriumcarbonat werden in einem 1 l Glasreaktor auf 5°C gebracht und anschliessend mit 217 g (1,1 Mol) PES-40, der 4 g (0,048 Mol) Natriumacetat zugesetzt wurde, durch Zutropfen bei dieser Temperatur zur Reaktion gebracht. Nach einer Reaktionszeit von 4 h wird in bekannter Weise aufgearbeitet. Lösemittel und nichtumgesetztes Trien werden über eine Kolonne abdestilliert.

Ergebnis:

158 g 1-Epoxycyclododecadien-5.9 mit einem Gehalt von 95%; dies entspricht einer Ausbeute von 84% d.Th., bezogen auf eingesetztes Cyclododecatrien-1.5.9.

**Patentansprüche**

1. Verfahren zur Herstellung von Epoxiden durch Epoxidation der zugrundeliegenden Olefine mit starke Säuren enthaltender Peressigsäure in Gegenwart von säurebindenden Mitteln in einem Lösungsmittel und/oder Überschuss des Olefins, dadurch gekennzeichnet, dass die starke Säure vor der Zugabe der Peressigsäure zu dem Reaktionsgemisch mit einer mindestens äquivalenten Menge einer anorganischen Base gebunden wird und dass das Reaktionsgemisch neben dem entsprechenden Olefin noch 0,50 bis 0,74 Val säurebindendes Mittel pro Mol Gesamtmenge an Peressigsäure und Essigsäure enthält.

2. Verfahren nach Anspruch 1, dadurch gekennzeichnet, dass man mit einer Lösung der Peressigsäure in der zugrundeliegenden Carbonsäure, die daneben noch eine starke Säure enthält, arbeitet.

3. Verfahren nach Anspruch 1 oder 2, dadurch gekennzeichnet, dass man eine Gleichgewichts-Peressigsäure, die neben der Peressigsäure noch Essigsäure und katalytische Mengen an starker Säure enthält, verwendet.

4. Verfahren nach Anspruch 3, dadurch gekennzeichnet, dass man Gleichgewichtsperessigsäure verwendet.

5. Verfahren nach einem der Ansprüche 1 bis 4, dadurch gekennzeichnet, dass man als säurebindende Mittel Alkalicarbonate oder Alkalihydrogencarbonate verwendet.

6. Verfahren nach einem der Ansprüche 1 bis 5, dadurch gekennzeichnet, dass man als Lösungsmittel Chlorkohlenwasserstoffe verwendet.

7. Verfahren nach einem der Ansprüche 1 bis 6, dadurch gekennzeichnet, dass man als anorganische Base zur Bindung der starken Säure die Alkali-oder Erdalkalisalze der jeweiligen Carbonsäure oder Carbonate, Hydrogencarbonate, Oxide oder Hydroxide der Alkali- oder Erdalkalimetalle verwendet.

8. Verfahren nach Anspruch 7, dadurch gekennzeichnet, dass man Natriumacetat als Base verwendet.

9. Verfahren nach einem der Ansprüche 1 bis 8, dadurch gekennzeichnet, dass die anorganische Base in fester Form oder als konzentrierte wässrige Lösung eingesetzt wird.

10. Verfahren nach einem der Ansprüche 1 bis 9, dadurch gekennzeichnet, dass ein bei der Zugabe der anorganischen Base zur Lösung der Peressigsäure gebildeter Niederschlag durch Zufügen von Wasser in Lösung gebracht wird.

11. Verfahren nach einem der Ansprüche 1 bis 10, dadurch gekennzeichnet, dass man der Peressigsäure oder/und dem Reaktionsgemisch Peroxidstabilisatoren zusetzt, insbesondere aus der Reihe der als Peroxidstabilisatoren bekannten Chelatbildner.

12. Verfahren nach einem der Ansprüche 1 bis 11, dadurch gekennzeichnet, dass man zwischen −20 und 60°C arbeitet.

13. Verfahren nach einem der Ansprüche 1 bis 12, dadurch gekennzeichnet, dass man zwischen 10 und 40°C arbeitet.

**Claims**

1. Method of producing epoxides by epoxidation of the basic olefins with peracetic acid containing strong acids in presence of an acid-binder in a solvent and/or olefin excess, characterized in that said strong acid is bound with an at least equivalent quantity of an inorganic base before adding said peracetic acid to the reaction mixture and that said reaction mixture, besides the corresponding olefin, further contains 0.50 to 0.74 Val of acid-binders per mol of the total quantity of peracetic acid and acetic acid.

2. Method of claim 1, characterized by operating with a solution of peracetic acid in the basic carboxylic acid, further containing a strong acid.

3. Method of claim 1 or 2, characterized by using an equilibrium peracetic acid, containing acetic acid and catalytic quantities of strong acid besides the peracetic acid.

4. Method of claim 3, characterized by using equilibrium peracetic acid.

5. Method of one of claims 1 to 4, characterized by using alkali carbonates or alkalihydrogencarbonates as acid-binders.

6. Method of one of claims 1 to 5, characterized by using chlorinated hydrocarbons as solvent.

7. Method of one of claims 1 to 6, characterized by using as inorganic base for binding the strong acid the alkali- or alkaline earth salts of the corresponding carboxylic acid or carbonates, hydrogen carbonates, oxides or hydroxides of the alkali- or alkaline earth metals.

8. Method of claim 7, characterized by using sodium acetate as base.

9. Method of one of claims 1 to 8, characterized by using the inorganic base in solid form or as concentrated aqueous solution.

10. Method of one of claims 1 to 9, characterized in that a deposit, formed when adding the anor-

ganic base to the solution of the peracetic acid, is dissolved by adding water.

11. Method of one of claims 1 to 10, characterized in that peroxide-stabilizers are added to the peracetic acid or/and the reaction mixture, in particular of the series of chelate formers known as peroxide stabilizers.

12. Method of one of claims 1 to 11, characterized in that the temperature lies between −20° and 60 °C.

13. Method of one of claims 1 to 12, characterized in that the temperature lies between 10° and 40 °C.

**Revendications**

1. Procédé de produire des époxydes par époxidation des oléfines à la base avec de l'acide peracétique contenant des acides forts, en présence de liants d'acides dans un solvant et/ou un surplus d'oléfines, caractérisé en ce que l'acide fort, avant l'addition de l'acide peracétique au mélange réactionnel, est lié d'une quantité au moins équivalente d'une base inorganique et que le mélange réactionnel, en plus de l'oléfine correspondante, contient encore 0,50 à 0,74 Val du liant d'acide acétique.

2. Procédé selon la revendication 1, caractérisé en ce qu'on travaille avec une solution de l'acide peracétique dans l'acide carboxylique à la base, en outre contenant un acide fort.

3. Procédé selon la revendication 1 ou 2, caractérisé en ce qu'on utilise un acide peracétique d'équilibre, contenant outre l'acide peracétique aussi de l'acide acétique et des quantités catalytiques de l'acide fort.

4. Procédé selon la revendication 3, caractérisé en ce qu'on utilise de l'acide peracétique d'équilibre.

5. Procédé selon l'une des revendications 1 à 4, caractérisé en ce qu'on utilise comme liants d'acide des carbonates alcalins ou des carbonates d'hydrogène alcalins.

6. Procédé selon l'une des revendications 1 à 5, caractérisé en ce qu'on utilise des hydrocarbures chlorés comme solvant.

7. Procédé selon l'une des revendications 1 à 6, caractérisé en ce qu'on utilise comme base inorganique, pour lier l'acide fort, les sels alcalins ou les sels alcalino-terreux de l'acide carboxylique ou des carbonates correspondants, des carbonates d'hydrogène, des oxydes ou hydroxydes des métaux alcalins ou des métaux alcalino-terreux.

8. Procédé selon la revendication 7, caractérisé en ce qu'on utilise de l'acétate de sodium comme base.

9. Procédé selon l'une des revendications 1 à 8, caractérisé en ce qu'on utilise la base inorganique en forme solide ou en solution aqueuse concentrée.

10. Procédé selon l'une des revendications 1 à 9, caractérisé en ce qu'un dépôt, formé à l'addition de la base inorganique à la solution de l'acide peracétique, est dissous en additionnant de l'eau.

11. Procédé selon l'une des revendications 1 à 10, caractérisé en ce qu'on additionne des stabilisants de peroxide à l'acide peracétique ou/et au mélange réactionnel, particulièrement de la série des chélatificateurs connus comme stabilisants de peroxide.

12. Procédé selon l'une des revendications 1 à 11, caractérisé en ce qu'on travaille à une température de −20° à 60 °C.

13. Procédé selon l'une des revendications 1 à 12, caractérisé en ce qu'on travaille à une température de 10° à 40 °C.